(19) Europäisches Patentamt
European Patent Office
Office européen des brevets

(11) EP 0 713 871 B1

(12) **EUROPÄISCHE PATENTSCHRIFT**

(45) Veröffentlichungstag und Bekanntmachung des Hinweises auf die Patenterteilung:
07.10.1998 Patentblatt 1998/41

(51) Int. Cl.$^6$: **C07D 231/12**, C08G 18/80

(21) Anmeldenummer: 95117656.9

(22) Anmeldetag: 09.11.1995

(54) **Verfahren zur Herstellung von 3,5-Dimethylpyrazol-blockierten Polyisocyanaten**

Process for the preparation of 3,5-dimethylpyrazol blocked polyisocyanates

Procédé pour la préparation de polyisocyanates masqués par 3,5-diméthylpyrazole

(84) Benannte Vertragsstaaten:
DE ES FR GB IT

(30) Priorität: 22.11.1994 DE 4441418

(43) Veröffentlichungstag der Anmeldung:
29.05.1996 Patentblatt 1996/22

(73) Patentinhaber: BAYER AG
51368 Leverkusen (DE)

(72) Erfinder:
• König, Eberhard, Dr.
D-51375 Leverkusen (DE)
• Engbert, Theodor, Dr.
D-50968 Köln (DE)

(56) Entgegenhaltungen:
EP-A- 0 159 117        EP-A- 0 500 495

**Beschreibung**

Die Erfindung betrifft ein neues Verfahren zur Herstellung von zumindest teilweise mit 3,5-Dimethylpyrazol (DMP) blockierten Polyisocyanaten unter Verwendung von in situ aus Acetylaceton und Hydrazinhydrat hergestelltem DMP.

Die Verwendung von DMP als Blockierungsmittel für Isocyanate ist bekannt (US-PS 3 248 398 bzw. EP-A-0 159 117). Mit DMP blockierte Lackpolyisocyanate können als Vernetzer in hochwertigen Einkomponenten-Polyurethan-Lackbindemitteln verwendet werden. Der Vorteil von DMP gegenüber anderen Blockierungsmitteln wie beispielsweise Butanonoxim liegt in einer wesentlich niedrigeren Thermovergilbung der Lacke und einer vergleichsweise niedrigen Einbrenntemperatur von ca. 130°C.

DMP entsteht durch Kondensation von Acetylaceton mit Hydrazin und stellt einen oberhalb 100°C schmelzenden Feststoff dar. Aus diesem Grund war bislang die großtechnische Handhabung immer mit einem größeren Aufwand verbunden als dies beispielsweise bei Verwendung von flüssigen Blockierungsmitteln der Fall ist. Es war daher die der Erfindung zugrundeliegende Aufgabe, ein neues Verfahren zur Herstellung von mit DMP blockierten Polyisocyanaten zur Verfügung zu stellen, bei welchem die isolierte Herstellung des Blockierungsmittels und dessen Handhabung als Feststoff entfällt.

Diese Aufgabe konnte überraschenderweise mit dem nachstehend näher beschriebenen erfindungsgemäßen Verfahren gelöst werden. Es hat sich nämlich gezeigt, daß das in organischer Lösung durch eine Kondensationsreaktion aus Acetylaceton und Hydrazinhydrat hergestellte Rohprodukt ohne Reindarstellung des vorliegenden DMP unmittelbar zur Blockierungsreaktion für organische Polyisocyanate verwendet werden kann, ohne das dies eine nennenswerte Beeinträchtigung der Qualität des so erhaltenen blockierten Polyisocyanats zur Folge hätte. Ein weiterer Vorteil des nachstehend näher beschriebenen erfindungsgemäßen Verfahrens ist in dem Umstand zu sehen, daß die Ausbeute an blockiertem Polyisocyanat, bezogen auf die eingesetzten Ausgangsmaterialien nahezu quantitativ ist, während die bislang praktizierte, vorab durchgeführte Reindarstellung von DMP und dessen anschließende Verwendung als Blockierungsmittel zwangsläufig zu nicht vermeidbaren Ausbeuteverlusten führt.

Gegenstand der Erfindung ist ein Verfahren zur Blockierung zumindest eines Teils der Isocyanatgruppen eines organischen Polyisocyanats mit 3,5-Dimethylpyrazol, dadurch gekennzeichnet, daß man in einer ersten Reaktionsstufe ein äquimolares Reaktionsgemisch aus Acetylaceton und Hydrazinhydrat in einem mit Wasser nicht mischbaren Lösungsmittel unter Abspaltung und Eliminierung des eingebrachten und des gebildeten Wassers einer Kondensationsreaktion unterzieht und in einer zweiten Reaktionsstufe die hierbei erhaltene organische Phase ohne weitere Aufarbeitung mit dem gegebenenfalls in einem Lösungsmittel gelöst vorliegenden Polyisocyanat unter zumindest teilweiser Blockierung von dessen Isocyanatgruppen mit dem in situ hergestellten 3,5-Dimethylpyrazol zur Reaktion bringt.

Bei den beim erfindungsgemäßen Verfahren zu blockierenden Polyisocyanaten handelt es sich um beliebige organische Polyisocyanate mit mindestens zwei (cyclo)aliphatisch oder aromatisch gebundenen Isocyanatgruppen. Beispielhaft genannt seien die bekannten Diisocyanate der Polyurethanchemie wie beispielsweise 2,4- und/oder 2,6-Diisocyanatotoluol (TDI), Hexamethylendiisocyanat (HDI), 1-Isocyanato-3,3,5-trimethyl-5-isocyanatomethyl-cyclohexan (IPDI), 4,4'-Diisocyanatodicyclohexylmethan (HMDI) und insbesondere die sich von diesen Diisocyanaten ableitenden Isocyanurat-, Urethan-, Allophanat-, Biuret- und/oder Uretdiongruppen aufweisenden Polyisocyanate. Auch längerkettige NCO-Prepolymere auf Basis der genannten Di- und/oder Polyisocyanate können beim erfindungsgemäßen Verfahren eingesetzt werden. Die bevorzugten beim erfindungsgemäßen Verfahren einzusetzenden Di- bzw. Polyisocyanate weisen ein Molekulargewicht von 168 bis 1000 und einen NCO-Gehalt von 15 bis 50 Gew.-% auf. Besonders bevorzugt werden beim erfindungsgemäßen Verfahren Isocyanuratgruppen aufweisende Derivate von HDI, IPDI und/oder HMDI eingesetzt.

Die erste Stufe des erfindungsgemäßen Verfahrens besteht darin, Acetylaceton (Pentan-1,3-dion) und Hydrazin (welches in der Regel als Hydrazinmonohydrat eingesetzt wird) in einem mit Wasser nicht mischbaren Lösungsmittel im Sinne einer Kondensationsreaktion umzusetzen. Als Lösungsmittel kommen beispielsweise Toluol, Xylol, 2-Methoxypropylacetat, Solventnaphtha 100 oder Gemische dieser Lösungsmittel in Betracht. Die Kondensationsreaktion erfolgt im allgemeinen innerhalb des Temperaturbereichs von 50 bis 150, vorzugsweise 70 bis 110°C, wobei pro Liter des genannten Lösungsmittels jeweils 4 bis 7, vorzugsweise 5 bis 6 Mol der beiden Ausgangskomponenten zum Einsatz gelangen. Die exotherme Reaktion ist nach Abscheidung des mit dem Hydrazinhydrat eingetragenen und des sich während der Kondensationsreaktion gebildeten Wassers beendet. Nach Abtrennung des abgeschiedenen Wassers wird die vorliegende organische Lösung direkt in der zweiten Stufe des erfindungsgemäßen Verfahrens eingesetzt. Hierzu wird die eine der beiden Reaktivkomponenten (entweder das in einem inerten Lösungsmittel wie beispielsweise Methoxypropylacetat gelöste Polyisocyanat oder die vorstehend genannte, in situ hergestellte Lösung des Blockierungsmittels) vorgelegt und die andere Komponente unter Durchmischen des Ansatzes hinzugegeben. Diese Blockierungsreaktion erfolgt im allgemeinen bei 50 bis 110, vorzugsweise 70 bis 90°C. Die Mengen der bei dieser zweiten Stufe des erfindungsgemäßen Verfahrens eingesetzten Reaktionspartner werden so gewählt, daß pro Äquivalent an unblockierten Isocyanatgruppen 0,3 bis 1,05 Mol des in der ersten Stufe des erfindungsgemäßen Verfahrens in situ gebildeten DMP zur Verfügung stehen. Falls blockierte Polyisocyanate hergestellt werden sollen, in denen lediglich DMP als Blockierungsmittel vorliegt, werden in der zweiten Stufe des erfindungsgemäßen Verfahrens pro Äquivalent an zu blockierenden Isocyanatgruppen 0,95 bis 1,05 Mol des in der ersten Stufe in situ gebildeten DMP eingesetzt. Vorzugsweise wird in diesem Fall mindestens eine äquivalente Menge des Blockierungsmittels eingesetzt. Der Verlauf der Reaktion kann an der Abnahme des NCO-Gehalts verfolgt werden.

Das erfindungsgemäße Verfahren eignet sich auch sehr gut zur Herstellung von mischblockierten Polyisocyanaten, d.h. von Polyisocyanaten, deren Isocyanatgruppen mit unterschiedlichen Blockierungsmitteln blockiert sind. Bei der Herstellung von solchen mischblockierten Polyisocyanaten wird neben DMP mindestens ein anderes Blockierungsmittel eingesetzt. Geeignete andere Blockierungsmittel sind beispielsweise Butanonoxim, 1,2,4-Triazol, Diisopropylamin, Malonsäureethylester oder Acetessigsäureethylester.

Bei der Herstellung von mischblockierten Polyisocyanaten nach dem erfindungsgemäßen Verfahren werden pro Äquivalent an Isocyanatgruppen des unblockierten Polyisocyanats mindestens 0,3 Mol, vorzugsweise 0,3 bis 0,9 Mol und besonders bevorzugt 0,4 bis 0,5 Mol DMP eingesetzt.

Zur Herstellung von mischblockierten Polyisocyanaten kann nach zwei unterschiedlichen Varianten verfahren werden:

Die erste Variante besteht darin, in der zweiten Stufe des erfindungsgemäße Verfahrens bereits mit mindestens einem "anderen" Blockierungsmittel teilblockierte Isocyanate einzusetzen, in welchem Falle die Menge des in situ vorab hergestellten DMP so bemessen wird, daß pro Äquivalent an noch vorliegenden freien Isocyanatgruppen 0,95 bis 1,05 Mol an vorab in situ hergestelltem DMP vorliegen. Hieraus und aus den oben gemachten Angaben ergibt sich, daß die Menge des "anderen" Blockierungsmittels bei bis zu 75 Äquivalentprozent, bezogen auf die Isocyanatgruppen des unblockierten Polyisocyanats liegen kann.

Gemäß der zweiten Variante der Herstellung von mischblockierten Polyisocyanaten werden in der zweiten Stufe des erfindungsgemäßen Verfahrens unblockierte Ausgangspolyisocyanate und eine unteräquivalente Menge des vorab in situ hergestellten DMP, bezogen auf die vorliegenden freien Isocyanatgruppen, eingesetzt. In diesem Falle beträgt die Menge des eingesetzten DMP jedoch, entsprechend den oben gemachten Ausführungen, mindestens 0,3 Mol pro Äquivalent an freien Isocyanatgruppen. Im Anschluß an die so bewirkte Teilblockietung des Ausgangspolyisocyanats erfolgt dann die Blockierung mit dem "anderen" Blockierungsmittel in an sich bekannter Weise. Die Menge des "anderen" Blockierungsmittels wird hier ebenfalls so bemessen, daß pro Äquivalent an noch vorliegenden freien Isocyanatgruppen 0,95 bis 1,05 Mol des "anderen" Blockierungsmittels vorliegen.

Die nach dem erfindungsgemäßen Verfahren hergestellten blockierten Polyisocyanate stellen wertvolle Ausgangsmaterialien zur Herstellung von Einkomponenten-Polyurethan-Lacken für die unterschiedlichsten hitzeresistenten Substrate dar. Besonders bevorzugte Reaktionspartner für die erfindungsgemäße Verfahrensprodukte sind die an sich bekannten Hydroxylgruppen aufweisenden Polyacrylatharze.

In den nachfolgenden Beispielen beziehen sich alle Prozentangaben auf das Gewicht.

## Beispiel 1 (erfindungsgemäß)

Ansatz:

| | |
|---|---|
| 85,0 g (0,85 Mol) | Acetylaceton |
| 42,5 g (0,85 Mol) | Hydrazinhydrat |
| 170,0 g (0,85 NCO-Val) | Isocyanuratgruppen aufweisendes Polyisocyanat auf Basis von 1,6-Diisocyanatohexan, NCO-Gehalt: 21 % |
| 100,0g | Xylol |
| 70,0 g | 2-Methoxy-propylacetat |
| 467,5 g | |
| -45,9 g (2 ,55 Mol) | Wasser |
| 421,6 g | (0,85 Val block. NCO-Gruppen), block. NCO-Gehalt: 8,4 %, Festkörpergehalt 60 %, Viskosität bei 22°C: 225 mPa.s |

Durchführung:

Stufe 1: Herstellung der 3,5-Dimethylpyrazol-Lösung

In einer Rührapparatur mit Wasserabscheider, Rückflußkühler und Tropftrichter werden Acetylaceton und die beiden Lösemittel vorgelegt und auf 70°C erwärmt. Hierzu tropft man Hydrazinhydrat, wobei sich die Temperatur der Reaktionsmischung auf 90 bis 100°C, also schwachem Rückfluß, erwärmt. Nach der Hydrazinhydratzugabe erwärmt man in dem Maße, daß ein ständiger Rückfluß vorhanden ist (Sumpftemperatur 120 bis 140°C). Nach ca. 30 min haben sich im Wasserabscheider 45 ml Wasser angesammelt. Die klare, fast farblose Reaktionslösung wird auf ca. 70°C abgekühlt und in Stufe 2 weiterverarbeitet.

Stufe 2: Herstellung des blockierten Polyisocyanates

Das flüssige Polyisocyanat auf Basis von 1,6-Diisocyanatohexan (Visk. bei 23°C ca. 3000 mPa.s) wird vorgelegt, auf ca. 70°C erwärmt und unter Rühren portionsweise mit der Reaktionslösung aus Stufe 2 versetzt. Nach beendeter Zugabe setzt man noch 1 Stunde bei 100°C um. Danach ist IR-spektroskopisch kein NCO-Gehalt mehr feststellbar.

Man erhält eine fast farblose, 60 %ige Lösung eines blockierten Polyisocyanates mit einem blockierten NCO-Gehalt von 8,4 % und einer Viskosität bei 22°C von 225 mPa.s. Ausbeute: 100 %, bezogen auf eingesetztes Polyisocyanat.

__Beispiel 2__ (erfindungsgemäß)

Ansatz:

| | |
|---|---|
| 85,0 g (0,85 Mol) | Acetylaceton |
| 42,5 g (0,85 Mol) | Hydrazinhydrat |
| 79,4 g (1,15 Mol) | 1,2,4-Triazol |
| 700,0 g (2,0 Val) | Isocyanuratgruppen aufweisendes Polyisocyanat auf Basis von Isophorondiisocyanat (IPDI), 70 %ig in 2-Methoxypropylacetat/Xylol (1/1), NCO-Gehalt: 12,0 % |
| 124,0 g | 2-Methoxypropylacetat |
| 100,0 g | Xylol |
| 1130,9 g | |
| - 45,9 g (2,55 Mol) | Wasser |
| 1085,0 g (2,0 Val) | blockierte NCO-Gruppen, block. NCO-Gehalt: 7,7 %, Festkörpergehalt: 60 %, Viskosität bei 22°C 8000 mPa.s |

Durchführung:

Stufe 1: Herstellung der 3,5-Dimethylpyrazol-Lösung

In einer Rührapparatur mit Wasserabscheider, Rückflußkühler und Tropftrichter werden Acetylaceton, Xylol und eine Teilmenge von 70 g Methoxypropylacetat vorgelegt und auf 70°C erwärmt. Hierzu tropft man Hydrazinhydrat, wobei sich die Temperatur der Reaktionsmischung auf 90 bis 100°C, also schwachen Rückfluß, erwärmt. Nach der Hydrazinhydratzugabe erwärmt man in dem Maße, daß ein ständiger Rückfluß vorhanden ist (Sumpftemperatur 120 bis 140°C). Nach ca. 30 min haben sich im Wasserabscheider 45 ml Wasser angesammelt. Die klare, fast farblose Reaktionslösung wird auf ca. 70°C abgekühlt und in Stufe 2 weiterverarbeitet.

Stufe 2: Herstellung des blockierten Polyisocyanates

Das obige IPDI-Trimerisat und eine Teilmenge Methoxypropylacetat von 54 g werden vorgelegt und auf 60°C erwärmt. Zu dieser gerührten Lösung gibt man die weiß-kristalline Schuppenwaren von 1,2,4-Triazol und erwärmt stufenweise auf 100 bis 105°C, wobei 1,2,4-Triazol in Lösung geht. Eine signifikante Exothermie kann hierbei nicht beobachtet werden. Nach einer Umsetzung von 30 Minuten bei 100 bis 105°C wird ein NCO-Gehalt von 4,3 % (berechnet sind 4,2 %) gemessen. Man läßt auf ca. 70°C abkühlen und gibt die ca. 70°C warme Lösung an 3,5-Dimethylpyrazol aus Stufe 1 portionsweise hinzu. Man erwärmt auf 100°C und setzt bei dieser Temperatur um, bis kein NCO-Gehalt IR-spektroskopisch mehr nachgewiesen wird (ca. 1 Stunde).

Man erhält eine blaß-gelbe Lösung eines mit sowohl Triazol als auch Dimethylpyrazol mischblockierten Polyisocyanates für die Verwendung in vergilbungsresistenten 1K-Klarlacken. Der BNCO-Gehalt dieser Lösung beträgt 7,7 % (berechnet), der Festkörpergehalt 60 % (berechnet) und die Viskosität bei 22°C ca. 8000 mPa.s. Ausbeute: 100 %, bezogen auf eingesetztes Polyisocyanat.

**Patentansprüche**

1. Verfahren zur Blockierung zumindest eines Teils der Isocyanatgruppen eines organischen Polyisocyanats mit 3,5-Dimethylpyrazol, dadurch gekennzeichnet, daß man in einer ersten Reaktionsstufe ein äquimolares Reaktionsgemisch aus Acetylaceton und Hydrazinhydrat in einem mit Wasser nicht mischbaren Lösungsmittel unter Abspaltung und Eliminierung des eingebrachten und des gebildeten Wassers einer Kondensationsreaktion unterzieht und in einer zweiten Reaktionsstufe die hierbei erhaltene organische Phase ohne weitere Aufarbeitung mit dem gegebenenfalls in einem Lösungsmittel gelöst vorliegenden Polyisocyanat unter zumindest teilweiser Blockierung von dessen Isocyanatgruppen mit dem in situ hergestellten 3,5-Dimethylpyrazol zur Reaktion bringt.

2. Verfahren gemäß Anspruch 1, dadurch gekennzeichnet, daß man in der zweiten Reaktionsstufe pro Äquivalent an Isocyanatgruppen des zumindest teilweise mit 3,5-Dimethylpyrazol zu blockierenden Polyisocyanats 0,95 bis 1,05 Mol des in situ in der ersten Stufe hergestellten Umsetzungsprodukts verwendet.

3. Verfahren gemäß Anspruch 1 und 2, dadurch gekennzeichnet, daß man als zumindest teilweise mit 3,5-Dimethylpyrazol zu blockierendes Polyisocyanat ein mit einem anderen Blockierungsmittel teilblockiertes Polyisocyanat verwendet, mit der Maßgabe, daß die Gesamtmenge der dann zur Blockierung des Polyisocyanats verwendeten Blockierungsmittel bei mindestens 95 Äquivalent-% und die Menge des 3,5-Dimethylpyrazols bei 40 bis 50 Äquivalent-%, jeweils bezogen auf die Isocyanatgruppen des unblockierten Polyisocyanats, liegen.

4. Verfahren gemäß Anspruch 1 und 2, dadurch gekennzeichnet, daß man als zumindest teilweise mit 3,5-Dimethylpyrazol zu blockierendes Polyisocyanat ein unblockiertes Polyisocyanat einsetzt, dieses in der zweiten Reaktionsstufe mit 0,4 bis 0,5 Mol pro Äquivalent an Isocyanatgruppen des in der ersten Reaktionsstufe in situ hergestellten Blockierungsmittels unter Blockierung eines Teils der Isocyanatgruppen umsetzt und das teilblockierte Polyisocyanat anschließend mit einem anderen Blockierungsmittel zumindest zu 95 Äquivalent-% blockiert.

5. Verfahren gemäß Anspruch 3 und 4, dadurch gekennzeichnet, daß man als anderes Blockierungsmittel Butanonoxim, 1,2,4-Triazol, Diisopropylamin, Malonsäurediethylester und/oder Acetessigsäureethylester verwendet.

6. Verfahren gemäß Anspruch 1 bis 5, dadurch gekennzeichnet, daß man als zumindest teilweise mit 3,5-Dimethylpyrazol zu blockierende Polyisocyanate organische Polyisocyanate des Molekulargewichtsbereichs 168 bis 1000 mit einem NCO-Gehalt von 15 bis 50 Gew.-% verwendet.

7. Verfahren gemäß Anspruch 1 bis 6, dadurch gekennzeichnet, daß man als zumindest teilweise mit 3,5-Dimethylpyrazol zu blockierende Polyisocyanate Isocyanuratgruppen aufweisende Lackpolyisocyanate auf Basis von 1,6-Diisocyanatohexan, 1-Isocyanato-3,3,5-trimethyl-5-isocyanato-methyl-cyclohexan und/oder 4,4'-Diisocyanato-dicyclohexylmethan verwendet.

## Claims

1. Process for blocking at least a proportion of the isocyanate groups of an organic polyisocyanate with 3,5-dimethylpyrazole, characterised in that in a first reaction stage an equimolar reaction mixture of acetylacetone and hydrazine hydrate is subjected to a condensation reaction in a water-immiscible solvent with cleavage and elimination of the introduced and formed water and in a second reaction stage the resultant organic phase is reacted without further working up with the polyisocyanate, which is optionally dissolved in a solvent, with at least partial blocking of the isocyanate groups thereof with the 3,5-dimethylpyrazole produced *in situ*.

2. Process according to claim 1, characterised in that in the second reaction stage 0.95 to 1.05 mol of the reaction product formed *in situ* in the first stage are used per equivalent of isocyanate groups of the polyisocyanate to be at least partially blocked with 3,5-dimethylpyrazole.

3. Process according to claims 1 and 2, characterised in that a polyisocyanate partially blocked with another blocking agent is used as the polyisocyanate to be at least partially blocked with 3,5-dimethylpyrazole, providing that the total quantity of the blocking agent then used to block the polyisocyanate is at least 95 equivalent percent and the quantity of the 3,5-dimethylpyrazole is 40 to 50 equivalent percent, in each case relative to the isocyanate groups of the unblocked polyisocyanate.

4. Process according to claims 1 and 2, characterised in that an unblocked polyisocyanate is used as the polyisocyanate to be at least partially blocked with 3,5-dimethylpyrazole, this unblocked polyisocyanate is reacted in the second stage of the reaction with 0.4 to 0.5 mol of the blocking agent produced *in situ* in the first reaction stage per equivalent of isocyanate groups with blocking of a proportion of the isocyanate groups and the partially blocked polyisocyanate is then blocked to an extent of at least 95 equivalent percent with another blocking agent.

5. Process according to claims 3 and 4, characterised in that the other blocking agent used is butanone oxime, 1,2,4-triazole, diisopropylamine, diethyl malonate and/or ethyl acetoacetate.

6. Process according to claims 1 to 5, characterised in that organic polyisocyanates of the molecular weight range 168 to 1000 with an NCO content of from 15 to 50 wt.% are used as the polyisocyanates to be at least partially blocked with 3,5-dimethylpyrazole.

7. Process according to claims 1 to 6, characterised in that lacquer polyisocyanates having isocyanurate groups and based on 1,6-diisocyanatohexane, 1-isocyanato-3,3,5-trimethyl-5-isocyanatomethylcyclohexane and/or 4,4'-diisocyanatodicyclohexylmethane are used as the polyisocyanates to be at least partially blocked with 3,5-dimethylpyrazole.

## Revendications

1. Procédé pour le blocage d'au moins une partie des groupes isocyanate d'un polyisocyanate organique avec du 3,5-diméthylpyrazole, caractérisé en ce que l'on soumet dans une première étape de réaction à une réaction de condensation un mélange réactionnel équimolaire d'acétylacétone et d'hydrate d'hydrazine dans un solvant non miscible à l'eau avec dissociation et élimination de l'eau introduite et de l'eau formée et en ce que l'on fait réagir dans une seconde étape de réaction la phase organique ainsi obtenue sans autre traitement avec le polyisocyanate présent éventuellement dissous dans un solvant avec blocage au moins partiel de ses groupes isocyanate avec le 3,5-diméthylpyrazole préparé in situ.

2. Procédé selon la revendication 1, caractérisé en ce que l'on utilise dans la seconde étape de réaction de 0,95 à 1,05 mole du produit de réaction préparé in situ dans la première étape par équivalent en groupes isocyanate du polyisocyanate à bloquer au moins partiellement avec du 3,5-diméthylpyrazole.

3. Procédé selon la revendication 1 et 2, caractérisé en ce que l'on utilise comme polyisocyanate à bloquer au moins partiellement avec du 3,5-diméthylpyrazole un polyisocyanate partiellement bloqué avec un autre agent de blocage dans la mesure où la quantité totale de l'agent de blocage utilisé alors pour le blocage du polyisocyanate est d'au moins 95% en équivalent et que la quantité de 3,5-diméthylpyrazole est de 40 à 50% en équivalent rapportés à chaque fois aux groupes isocyanate du polyisocyanate non bloqué.

4. Procédé selon les revendications 1 et 2, caractérisé en ce que l'on utilise comme polyisocyanate à bloquer au moins partiellement avec du 3,5-diméthylpyrazole un polyisocyanate non bloqué, en ce que l'on fait réagir celui-ci dans la seconde étape de réaction avec de 0,4 à 0,5 mole par équivalent en groupes isocyanate de l'agent de blocage préparé in situ dans la première étape de réaction avec blocage d'une partie des groupes isocyanate et en ce que l'on bloque ensuite le polyisocyanate partiellement bloqué avec un autre agent de bloquage jusqu'au moins 95% en équivalent.

5. Procédé selon la revendication 3 et 4, caractérisé en ce que l'on utilise comme autre agent de bloquage le butanonoxime, le 1,2,4-triazole, la diisopropylamine, un ester diéthylique d'acide malonique et/ou un ester éthylique d'acide acétoacétique.

6. Procédé selon la revendication 1 à 5, caractérisé en ce que l'on utilise comme polyisocyanates à bloquer au moins partiellement avec du 3,5-diméthylpyrazole des polvisocyanates organiques du domaine de poids moléculaire de 168 à 1 000 avec une teneur en NCO de 15 à 50% en poids.

7. Procédé selon la revendication 1 à 6, caractérisé en ce que l'on utilise comme polyisocyanates à bloquer au moins partiellement avec du 3,5-diméthylpyrazole des polyisocyanates pour laques présentant des groupes isocyanurate à base de 1,6-diisocyanatohexane, de 1-isocyanato-3,3,5-triméthyl-5-isocyanatométhylcyclo-hexane et/ou de 4,4'-diisocyanato-dicyclohexylméthane.